# EUROPEAN PATENT APPLICATION

(11) **EP 3 422 226 A1**
(43) Date of publication of application: **02.01.2019**
(21) Application number: 17305824.9
(22) Date of filing: 29.06.2017
(51) Int. Cl.: G06F 19/00, A61B 8/08

(54) **DEVICE AND METHOD FOR PREDICTING AN UNFOLDED STATE OF A FOLDABLE IMPLANT IN CARDIOVASCULAR TISSUE**

(71) Applicant: Koninklijke Philips N.V., 5656 AE Eindhoven (NL)
(72) Inventor: WISSEL, Tobias, 5656 AE Eindhoven (NL); MORALES VARELA, Hernán Guillermo, 5656 AE Eindhoven (NL); GRASS, Michael, 5656 AE Eindhoven (NL)
(74) Representative: de Haan, Poul Erik

(57) **Abstract**

The present invention relates to a device for predicting an unfolded state of a foldable implant in cardiovascular tissue, the device (2) comprising: a receiving unit (20); a segmentation module (21); a search unit (22); a registration module (23); and a simulation unit (24); wherein the receiving unit (20) is configured to receive pre-treatment 3D planning image data and treatment image data, the treatment image data comprising catheter image data, the catheter image data comprising landmark image data; wherein the segmentation module (21) is configured to segment the pre-treatment 3D planning image data resulting in segmented 3D planning image data; wherein the search unit (22) is configured to locate the landmark image data within the treatment image data resulting in a known landmark position and a known landmark orientation; and wherein the registration module (23) is configured to register the segmented 3D planning image data to the treatment image data; wherein the simulation unit (24) is configured to simulate an unfolded state of a foldable implant in cardiovascular tissue based on the known landmark position and the known landmark orientation in the cardiovascular tissue resulting in a simulated unfolded implant positioned in cardiovascular tissue. The present invention provides the knowledge of the unfolded implant in the cardiovascular tissue as well as its interaction with the anatomical placement domain at the current landmark position prior to the real deployment.

## Description

### FIELD OF THE INVENTION

The present invention relates to a device and a method for predicting an unfolded state of a foldable implant in cardiovascular tissue.

### BACKGROUND OF THE INVENTION

Valvular heart diseases are treated by implanting artificial cardiac valves. Severe valve heart diseases facing high risk for surgery may be treated by inserting artificial cardiac valves through the vascular system. The insertion may for example be performed by a percutaneous pulmonary valve implantation (PPVI) or a transcatheter aortic valve implantation (TAVI). TAVI has rapidly evolved into a wide spread interventional option and is part of the current ECS/EACTS guidelines for treating aortic stenosis. TAVI is increasingly seen as relevant for low risk patients and has the potential to develop into a first-line treatment for most patients with aortic valve disease.

In addition, transcatheter mitral valve implantations (TMVI) are currently under investigation and have shown promising results for treating mitral regurgitation.

The planning of the treatment is typically based on 3D cardiac computed tomography angiography (CTA) data. The planning of the treatment further corresponds to the decision point for selection and sizing of the bioprothesis, i.e. the valve implant. During the intervention, the crimped implant is delivered via guided catheters along the access route and deployed at the landing zone under transesophageal echocardiography (TEE) and/or fluoroscopy guidance. The accurate placement of the implant is essential to avoid postoperative complications and a degraded treatment outcome.

Transcatheter treatment of valve diseases suffers from several complications. Many of them are directly or indirectly linked to the final implant selection, deployment and placement. Therefore, the placement result needs to be optimized with respect to manifold conditions and constraints. How exactly the relevance for each of those will manifest for a particular treatment is often strongly patient-specific.

In the well-studied case of TAVI, for instance, the implant is required to be slightly oversized for the target region to avoid paravalvular leakage after the implantation. On the other hand, if the implant applies too much stress to the surrounding tissue, this may entail the risk for ruptures or lead to a dysfunction of the cardiac conduction system. The latter is more relevant for some heart structures like the His bundle than for others. This in turn imposes constraints on the optimal placement location such as a restricted implantation depth into the left ventricular outflow tract (LVOT). Finally, the implant geometry after the deployment needs to spatially fit the anatomical morphology in the desired landing zone. This also relates to possibly asymmetrically distributed calcifications in the valve area, which can affect the deployment process. In addition, the coverage or obstruction of surrounding vessels such as the coronary arteries or outflow tracts must be taken into consideration.

Other valve replacements may suffer from similar complications. Examples include coronary compression after PPVI or LVOT obstruction after TMVI.

While all these factors can be considered during the offline planning process, it is often challenging to handle all of them online, i.e. during the intervention. The operator, i.e. the clinician, navigates the crimped implant with 2D angiography/fluoroscopy and/or TEE imaging to the desired landing zone. There, it must be judged from a certain catheter position, how a deployed implant would unfold into the surrounding anatomy and whether all important conditions will be matched.

This is particularly important in cases where the implant cannot be repositioned after deployment, i.e. if the deployment is irreversible, where further navigation becomes very difficult with a partially deployed implant, or where repositioning, if necessary, is an inconvenient and time-consuming process. Further difficulty arises when judging whether all conditions will be sufficiently met after deployment.

### SUMMARY OF THE INVENTION

There may thus be a need to know the position of the implant prior to the deployment.

Accordingly, a device may be provided for predicting an unfolded state of a foldable implant in cardiovascular tissue comprises: a receiving unit; a segmentation module; a search unit; a registration module; and a simulation unit; wherein the receiving unit is configured to receive pre-treatment 3D planning image data and treatment image data, the treatment image data comprising catheter image data, the catheter image data comprising landmark image data; wherein the segmentation module is configured to segment the pre-treatment 3D planning image data resulting in segmented 3D planning image data; wherein the search unit is configured to locate the landmark image data within the treatment image data resulting in a known landmark position and a known landmark orientation; and wherein the registration module is configured to register the segmented 3D planning image data to the treatment image data; wherein the simulation unit is configured to simulate an unfolding implant at the known landmark position and the known landmark orientation in the cardiovascular tissue resulting in a simulated unfolded implant positioned in cardiovascular tissue.

The device for predicting the unfolded state of a foldable implant in cardiovascular tissue receives pre-treatment 3D planning image data via the receiving unit. That data comprises 3D images of the patient's heart or further vascular system before the intervention. For further processing, the segmentation module segments the 3D planning image data into segmented 3D planning image data. Furthermore, the receiving unit receives treatment image data during the intervention, i.e. during the implantation process of the implant. The treatment image data comprises the catheter image data wherein the catheter image data comprises landmark image data.

The search unit searches for the landmark in the treatment image data comprising the catheter image data. Thus, the device locates the landmark and further determines the orientation of the landmark with the search unit. The registration module may further register the segmented 3D planning image data to the treatment image data. This means that the location and the orientation of the landmark being derived from the treatment image data is mapped to the segmented 3D planning image data. The landmark and the landmark orientation may be shown in the segmented 3D planning image data and further in the 3D planning image data. By knowing the position and the orientation of the landmark in the 3D planning image data, the device provides the knowledge of the position of the landmark and the catheter orientation prior to the deployment of an implant being arranged close to the landmark. By knowing the position and orientation of the landmark, the position and the orientation of the foldable implant in the folded state is also known. With that knowledge, the position and the orientation of the foldable implant in the unfolded state may be predicted. This provides the knowledge of the foldable implant in the unfolded state as well as its interaction with the anatomical placement domain at the current landmark position prior to the real deployment. The prediction of the unfolded state of the foldable implant therefore provides a prediction of the position and the orientation of the foldable implant in the unfolded state in the cardiovascular tissue as well as the dimension of the implant. The dimension of the foldable implant in the unfolded state determines the forces which the implant applies on the cardiovascular tissue. The simulation unit further simulates the unfolding of the foldable implant close to the landmark. The simulation allows an operator to determine how the foldable implant in the unfolded state is arranged in the cardiovascular tissue in the pre-treatment 3D cardiac planning image data. This means that the device simulates the unfolded state of the foldable implant at the catheter position in the cardiovascular tissue during the treatment before the real deployment of the implant, i.e. when the foldable implant is still in the folded state. This may for example be performed for each catheter position during the penetration process of the catheter into the cardiovascular tissue. The operator can simulate the placement result for the current initial position without actually executing it practically. This improves the prediction accuracy for the positioning of the implant in the cardiovascular tissue.

Alternatively or in addition, the landmark position and orientation may be provided directly in the 3D planning image data. In this case, an implant placement procedure can be planned in advance, without using live treatment image data. It follows that a search unit and registration unit, in this embodiment, may not be required.

Instead, for example, a user interface may be provided configured to allow an operator or physician to interact with the 3D planning image data and select the landmark position and orientation manually or in a (semi)automatic manner. For example, a user can navigate a catheter model through the 3D planning data and select a current catheter position and orientation as the landmark position and orientation to be used in the simulation. Thus, the device can be used in a planning phase, prior to an actual intervention, to check several different potential placement locations for the implant in the cardiovascular tissue.

According to a further example, the device further comprises: a feedback module; wherein the feedback module is configured to provide a visual feedback of the simulated unfolded implant positioned in the cardiovascular tissue.

The feedback module therefore directly provides visual and quantitative feedback, so that the operator can judge whether the simulated result will meet the needs or whether adjustments of the position of the implant, i.e. the landmark position and the landmark orientation, are required.

In an example, the registration module is implemented by a dedicated 3D/3D or 2D/3D inter-modality registration algorithm.

If in a first exemplary embodiment of the registration module similar modalities are used like e.g. CTA as pre-treatment 3D planning image data and e.g. fluoroscopy as treatment image data, the registration may be implemented based on minimizing a cost function that relates image and contrast features of corresponding scans in the pre-treatment 3D planning image data and the treatment image data.

In a second exemplary embodiment of the registration module, the registration module takes the segmented 3D planning image data e.g. CTA. Furthermore, the segmentation module applies a segmentation to the treatment image data being acquired by e.g. TEE, resulting in segmented treatment image data. Then the registration module is configured to register an output of the segmented pre-treatment 3D-planning image data and an output of the segmented treatment image data.

In a first example of the second exemplary embodiment, the registration may be performed using a volume bitmask labelling voxel by voxel of the image data with the segment it belongs to.

In a second example of the second exemplary embodiment, the registration may be performed with further processed segmented 3D-planning image data, i.e. not on image data level but on segmentation output level, which may be performed by determining e.g. 3D surface meshes of the anatomy being determined from the segmented 3D-planning image data and the treatment image data. After generating the surface or contour meshes of relevant structures (3D or 2D), the data is modality independent but patient specific and can be registered on this more abstract level.

In a third example of the second exemplary embodiment, a surface mesh in the 3D-planning image data and the treatment image data is generated. The segmentation of the 3D-planning image data and the treatment image data is performed based on the surface meshes. I.e. the surface meshes of the 3D-planning image data and the treatment image data may be converted into volume bitmasks.

In a third exemplary embodiment of the registration module, manual or automatic registration based on a set of natural landmarks and/or a set of artificial landmarks could be used. These can be obtained from the image data or also using alternatively the segmentation output e.g. labels on the mesh such as aortic valve nadir points. While the former can involve anatomical landmarks, the latter can include any kind of implanted fiducials.

In an example, the segmentation module is configured to segment the treatment image data resulting in segmented treatment image data.

In an example, the treatment image data may be 2D image data. In another example, the treatment data may be 3D image data. The treatment data is acquired during a treatment of a patient, i.e. during an implantation process. During the treatment, a catheter may be inserted into the patient's vascular system. The catheter may comprise a landmark. Close to the landmark, a foldable implant may be unfolded, i.e. the implant is transferred from a folded state into an unfolded state.

According to the present invention, also a system for predicting an unfolded state of a foldable implant in cardiovascular tissue comprises: a catheter; and a device according to one of the preceding claims; wherein the catheter comprises a landmark; and a foldable implant in a folded state; wherein the catheter is insertable in a patient's vascular system.

According to an example, the system further comprises: an image acquisition device; wherein the image acquisition device is configured to acquire treatment image data; and wherein the image acquisition device is configured to provide the treatment image data.

According to an example, the foldable implant may be a foldable cardiac valve implant.

In another example, the foldable implant may be a stent, particularly an abdominal aortic aneurysm (AAA) stent. The stent may be implanted in a patient's blood vessel of the cardiovascular system. Particularly, an AAA stent may be implanted in a patient's aorta.

According to the present invention, also a method for predicting an unfolded state of a foldable implant in cardiovascular tissue comprises the following steps: a) receiving pre-treatment 3D planning image data and treatment image data, the treatment image data comprising landmark image data with a receiving unit; b) receiving treatment image data with the receiving unit, the treatment image data comprising landmark image data; c) segmenting the pre-treatment 3D planning image data with a segmentation module resulting in segmented 3D planning image data; d) locating the landmark image data within the segmented treatment image data with a search unit resulting in a known landmark position and a known landmark orientation; and e) registering the segmented 3D planning image data to the acquired segmented treatment image data with a registration module; f) simulating an unfolding implant relative to the known landmark position and the known landmark orientation in the cardiovascular tissue with a simulation unit resulting in a foldable implant in an unfolded state positioned in cardiovascular tissue; and wherein step b) and c) may be performed in any order.

According to the present invention, also a computer program element for controlling an apparatus according to the above description which, when being executed by a processing unit, is adapted to perform the method steps according to the above description.

According to the present invention, also a computer readable medium has stored the program element according to the above description.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic drawing of the system for predicting the position of an unfolding implant in cardiovascular tissue.
Fig. 2 shows schematic drawing of a catheter with a foldable implant.
Fig. 3 shows a schematic drawing of the device for predicting the position of an unfolding implant in cardiovascular tissue with the patient.
Fig. 4 shows a schematic drawing of image data with simulated unfolded implants.
Fig. 5 shows a schematic flowchart of the method for predicting the position of an unfolding implant in cardiovascular tissue.

### DETAILED DESCRIPTION OF EMBODIMENTS

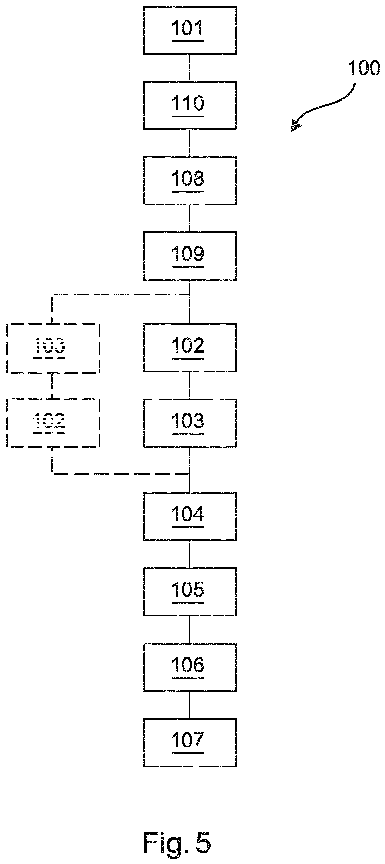

Before further describing the imaging system and the device, examples of a method are described in further detail referring to Fig. 5.

Fig. 5 shows the method 100 for predicting the position of an unfolding implant in cardiovascular tissue.

In step 101, pre-treatment 3D planning image data is received with a receiving unit. The pre-treatment 3D planning image data may be received prior to the treatment image data. In the following example, the implantation of a cardiac valve implant in a patient's heart is described. However, the implantation of further foldable implants on further locations of the cardiovascular system is analogous.

The pre-treatment 3D planning image data may be 3D computed tomography angiography data of the patient's heart and/or further vascular system prior to a surgery.

In step 110, a foldable cardiac valve implant is provided as foldable implant in a catheter. The catheter may be arranged in a patient's vascular system. This defines the patient's treatment.

In step 108, treatment image data with an image acquisition device is acquired during the patient's treatment. The image acquisition may be performed by an image acquisition device.

The acquired treatment image data may be provided by of the image acquisition device in step 109.

In step 102, treatment image data is received with the receiving unit. The treatment image data comprises catheter image data with landmark image data. The landmark may be e.g. the catheter tip. In this case, the catheter is shown having a catheter tip. This means that also a region close to a catheter tip, the region comprising a foldable implant in a folded state may be shown.

In further embodiments, the landmark may be any region of the catheter being close to the foldable implant in the folded state. Also, the foldable implant itself may be used as landmark.

In step 103 the pre-treatment 3D planning image data may be segmented into segmented 3D planning image data by a segmentation module. The segmentation of the pre-treatment 3D planning image data provides three-dimensional segments of the pre-treatment 3D planning image data. Those three-dimensional segments for the segmented 3D planning image data. Those segments stand for certain locations and positions in the depicted cardiovascular tissue.

In an example the segmentation may involve adapting shape-constrained deformable models to the 3D planning image. Those models may correspond to generic models of the cardiovascular tissue, which can be adapted to different imaging modalities to personalize a surface mesh for a specific patient. This personalized mesh will serve as the domain for the subsequent placement simulation.

Steps 102 and 103 may be performed in any order. In the embodiment described above, the treatment image data is received before segmenting the pre-treatment 3D planning image data.

In an alternative embodiment, the pre-treatment 3D planning image data may be segmented before the receiving of the treatment image data.

In step 104, the landmark image data within the treatment image data is located by a search unit. This step provides information about the position and the orientation of the landmark in the treatment image data. The catheter position and the catheter orientation are then known.

In step 105, the segmented 3D planning image data is registered to the treatment image data by a registration module. This means, that the segments of the pre-treatment 3D planning image data are linked to the treatment image data such that the known landmark position and the known landmark orientation being determined in the treatment image data is linked to the corresponding segments in the segmented 3D planning image data. Thus, with this step, the position and the orientation of the landmark may be met to the three-dimensional image of the cardiovascular tissue wherein the landmark has the correct orientation and position.

In step 106, based on the known landmark position and the known landmark orientation, the unfolding of the foldable implant close to the landmark is simulated with a simulation unit. In an example, a model of the unfolding implant interacts with a personalized surface mesh obtained during segmentation of the 3D planning date, which mesh represents the cardiovascular tissue surrounding the landmark position,

The result is a simulated foldable implant in an unfolded stated positioned in the cardiovascular tissue. An operator may then see how the unfolding process of the implant may influence the deployment of the implant. The simulation therefore delivers the final state of the foldable implant in the unfolded state in the cardiovascular tissue at the landmark position. Thus, prior to the actual deployment of the foldable implant in the cardiovascular tissue, the operator may simulate whether the implant is placed correctly and whether the effect of the implant on the surrounding cardiovascular tissue at the planned location does not harm the patient.

In an example, the simulation may provide one or more quantitative parameters representing potential complications caused by the unfolded implant. For example, relevant parameters may include stress distributions caused by the unfolding or unfolded device on the surrounding tissues, anatomical and device deformations, distances to relevant anatomical landmarks, implantation depth, or an indication of critical structures being obstructed by the unfolded device.

Step 107 provides a visual feedback of the simulated foldable implant positioned in the cardiovascular tissue with a feedback module. The feedback module may be a monitor device on which an operator may see the simulated foldable implant in the unfolded state in the cardiovascular tissue. The operator may assess the effect of the foldable implant in the unfolded state on the cardiovascular tissue. For instance, a visual feedback representing one or more relevant quantitative parameters provided by the simulation may be overlaid onto the planning computed tomography angiography data and be updated with the catheter position and the deployed device estimation over time. This may provide a real-time feedback for visual inspection by the operator.

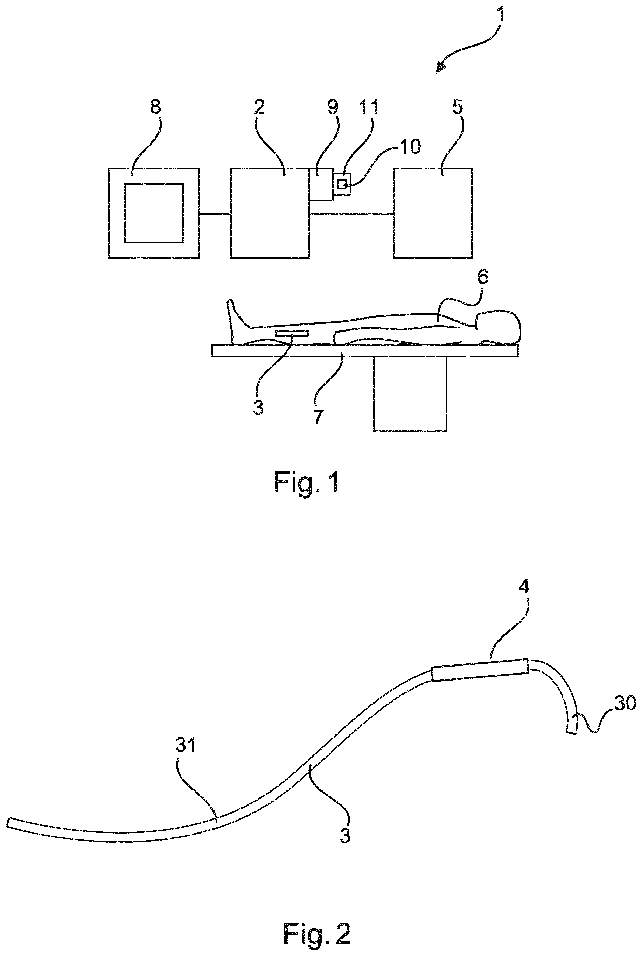

According to Fig. 1, the system 1 for predicting the unfolded state of a foldable implant in cardiovascular tissue comprises a device 2 for predicting the unfolded state of a foldable implant in cardiovascular tissue, a catheter 3 and an image acquisition device 5.

The image acquisition device 5 acquires treatment image data during the treatment of a patient with the catheter 3. The image acquisition device 5 may be a 2D angiography/fluoroscopy device. In an alternative embodiment of the image acquisition device 5 may be a TEE imaging device. A surgeon may use the image acquisition device 5 to navigate the catheter 3 through the patient's vascular system.

The patient 6 may be positioned on a support device 7. The catheter 3 may be introduced into the patient's vascular system in the patient's leg region. The image acquisition device 5 acquires treatment image data of the patient's cardiovascular tissue and may also provide images of further body parts of the patient 6 to navigate the catheter 3 through the vascular system of the patient 6.

The catheter 3 is further shown in Fig. 2. The catheter 3 comprises a landmark 30 and a main section 31. The landmark may be e.g. the catheter tip. In further embodiments, the landmark may be any region of the catheter being close to the foldable implant in the folded state. Also, the foldable implant itself may be used as landmark.

A foldable implant 4 is arranged close to the landmark 30 at the main section 31 of the catheter 3. The foldable implant 4 is in a folded state. In the folded state, the foldable implant substantially comprises the same diameter as the catheter 3. The foldable implant 4 may be unfolded in a patient's cardiovascular tissue. If the foldable implant 4 is unfolded, it is in an unfolded state.

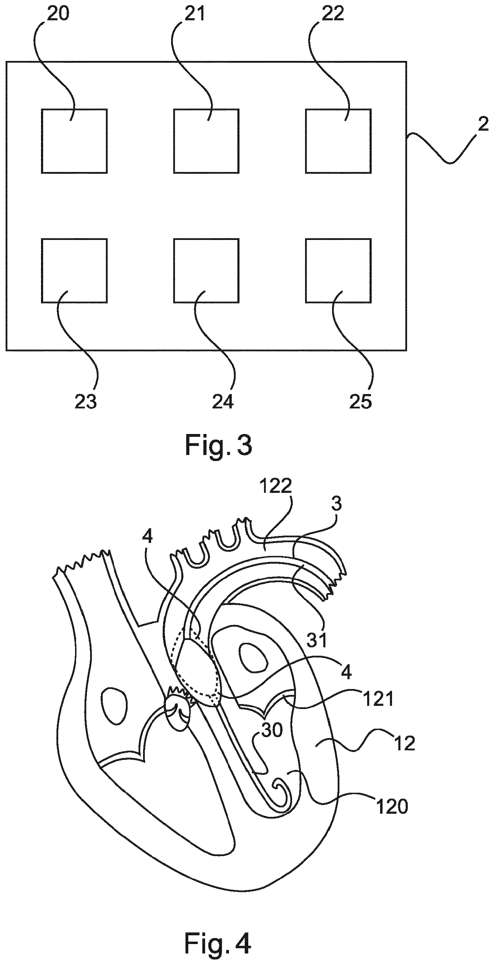

Referring now to Fig. 3, the device 2 for predicting the unfolded state of a foldable implant in cardiovascular tissue is described.

The device 2 for predicting the position of an unfolding implant in tissue comprises a receiving unit 20, the segmentation module 21, a search unit 22, a registration module 23, a simulation unit 24, and a feedback module 25.

The receiving unit 20 receives treatment image data which may be provided by the image acquisition device 5. Furthermore, the receiving unit 20 may receive pre-treatment 3D planning image data.

Furthermore, the acquired treatment image data may be transmitted from the image acquisition device 5 to the device 2. The receiving unit 20 then receives the treatment image data.

The segmentation module 21 may segment the pre-treatment 3D planning image data. The segmentation module 21 thus provides segmented 3D planning image data. The segments of the segmented 3D planning image data define regions of the pre-treatment 3D planning image data. As described in the above, for example, the segmentation module 21 may implement a segmentation algorithm based on shape-constrained deformable models being adapted to the 3D planning image data.

The treatment image data is searched by the search unit 22, wherein the search unit 22 locates the landmark image data within the treatment image data. The search unit 22 further determines the landmark position and the landmark orientation of the located landmark image data. Thus, the search unit 22 provides a known landmark position and a known landmark orientation of a catheter 3 being inserted into the patient 6.

The known landmark position and the known landmark orientation are registered to the segmented 3D planning image data by the registration module 23. The registration module 23 therefore links the known landmark position and the known landmark orientation from the treatment data to the pre-treatment 3D planning image data.

During the propagation of the catheter 3 in the patient's vascular system, the image acquisition device 5 may provide treatment image data for a plurality of positions of the catheter 3 in the patient's vascular system. This also applies for the patient's cardiovascular tissue.

The simulation unit 24 may simulate the unfolding of the foldable implant in the cardiovascular tissue based on the known landmark position and the known landmark orientation. The simulation unit 24 considers the known landmark orientation and the known landmark position in the cardiovascular tissue to simulate the placement of the unfolded implant in the cardiovascular tissue.

In an example, the simulation unit 24 uses a model which interacts with the anatomical information, e.g. surface meshes, obtained from the segmentation module 21. The simulation by the simulation unit 24 starts from the known catheter position in the pre-treatment 3D planning image data and evaluates how the implant would fit into the surrounding anatomy given the current situation. The evaluation may e.g. be performed with finite element methods (FEM), which simulate an entire biophysical interaction. In another example, the evaluation may be performed with simpler mass-spring models or deformable surface models. The latter two might be favorable in online situations as they are temporally less expensive. They correspond to heuristic approximations of the real interaction process. This is particularly true for the surface models, which carry certain properties and unfold into or affect the placement domain based on a minimization of internal and external energy terms.

The simulated unfolded implant in the cardiovascular tissue has been a determined simulated position which can be evaluated by an operator with the help of the feedback module 25. The feedback module 25 is the interface between the operator and the device 2. Furthermore, the feedback module 25 may provide the feedback information on a monitor device 8 providing visual feedback. An operator may extract the feedback information from the monitor device 8 and decide whether the simulation being provided by the device 2 fits to the patient's medical conditions.

The operator may assess the simulated implant position in the unfolded state and in the cardiovascular tissue. The feedback module may e.g. visualize one or more quantitative parameters obtained in the simulation by the simulation unit 24, such as the stress distributions on the surrounding tissues, anatomical and device the formation, distances to relevant anatomical landmarks, implantation depth or other measures which are specific to treatment complications.

The visual feedback of the result may be overlaid onto the planning computed tomography angiography data and be updated with the catheter position and the deployed device estimation over time. This may provide a real-time feedback for visual inspection by the operator. This visual feedback may be accompanied by the display of relevant quantitative measures such as, but not restricted to, implantation depth, tissue stress and deformation, obstruction of critical structures etc.

Fig. 4 shows an example image from simulated image data. The image comprises an image of the heart 12 having a heart valve 121 and the left main chamber 120. The exiting heart valve of the left main chamber 120 shall be replaced by the implant 4. The replacement may be a functional replacement, i.e. implant 4 is arranged on top of the exiting heart valve. The catheter 3 propagates through the aorta 122. The landmark 30 is in the left main chamber 120. The image of figure 3 shows two positions of the simulated implant 4. A first position is shown with dotted lines. In this simulated position of the simulated implant 4 is too far outside of the left main chamber 120. The operator therefore decided to further propagate the catheter 3 into the left main chamber 120. A new simulation then provides the full lined implant for in the second position.

If the foldable implant is a stent which shall be positioned in a blood vessel, the analogous procedure as described above is performed. The catheter 3 is then propagated to the position of the blood vessel through the vascular system. The pre-treatment 3D planning image data is then taken from the designated position of the implant in the blood vessel. Furthermore, the simulation is performed with the modelling of the blood vessel.

In another exemplary embodiment of the present invention, a computer program 10 or a computer program element is provided, that is characterized by being adapted to execute the method steps of the method according to one of the preceding embodiments, on an appropriate system.

The computer program element 10 might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor 9. The data processor 9 may thus be equipped to carry out the method 100 of the invention.

This exemplary embodiment of the invention covers both, a computer program that right from the beginning uses the invention and a computer program that by means of an up-date turns an existing program into a program that uses the invention.

Further on, the computer program element 10 might be able to provide all necessary steps to fulfil the procedure of an exemplary embodiment of the method 100 as described above.

According to a further exemplary embodiment of the present invention, a computer readable medium 11, such as a CD-ROM, is presented wherein the computer readable medium has a computer program element 10 stored on it which computer program element 10 is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device for predicting an unfolded state of a foldable implant in cardiovascular tissue, the device (2) comprising:
- a receiving unit (20);
- a segmentation module (21);
- a search unit (22);
- a registration module (23); and
- a simulation unit (24);
wherein the receiving unit (20) is configured to receive pre-treatment 3D planning image data and treatment image data, the treatment image data comprising catheter image data, the catheter image data comprising landmark image data;
wherein the segmentation module (21) is configured to segment the pre-treatment 3D planning image data resulting in segmented 3D planning image data;
wherein the search unit (22) is configured to locate the landmark image data within the treatment image data resulting in a known landmark position and a known landmark orientation;
wherein the registration module (23) is configured to register the segmented 3D planning image data to the treatment image data; and
wherein the simulation unit (24) is configured to simulate an unfolded state of a foldable implant in cardiovascular tissue based on the known landmark position and the known landmark orientation in the cardiovascular tissue resulting in a simulated unfolded implant positioned in cardiovascular tissue.

2. Device according to claim 1, wherein the device further comprises:
- a feedback module (25);
wherein the feedback module (25) is configured to provide a visual feedback of the simulated unfolded implant positioned in the cardiovascular tissue.

3. Device according to one of claims 1 or 2, wherein the registration module (23) is implemented by a dedicated 3D/3D or 2D/3D inter-modality registration algorithm.

4. Device according to any one of the preceding claims, wherein the registration module (23) is configured to minimize a cost function that relates image and contrast features of corresponding scans of similar modalities in the pre-treatment 3D planning image data and the treatment image data.

5. Device according to any one of the preceding claims, wherein the segmentation module (21) is configured to apply a segmentation to the treatment image data resulting in segmented treatment image data;
wherein the registration module (23) is configured to register an output of the segmented pre-treatment 3D-planning image data and an output of the segmented treatment image data.

6. Device according to any one of the preceding claims, wherein the registration module (23) is configured to register the segmented 3D planning image data to the treatment image data based on a set of natural landmarks and/or a set of artificial landmarks.

7. Device according to any one of the preceding claims, wherein the segmentation unit (21) is configured to adapting a shape-constrained deformable model to the 3D planning image so as to generate a patient-specific personalized surface mesh.

8. Device according to claim 7, wherein the simulation unit (24) is configured to carry out the simulation by modeling an interaction between the implant and the personalized surface mesh.

9. Device according to any one of the preceding claims, wherein the simulation unit (24) is configured to provide one or more quantitative parameters representing potential complications caused by the unfolded implant.

10. A system for predicting an unfolded state of a foldable implant in cardiovascular tissue, the system (1) comprising:
- a catheter (3); and
- a device (2) according to one of the preceding claims;
wherein the catheter comprises
- at least one landmark (30); and
- a foldable implant (4) in a folded state;
wherein the catheter (3) is insertable in a patient's vascular system.

11. System according to claim 10, wherein the system further comprises:
- an image acquisition device (5);
wherein the image acquisition device (5) is configured to acquire treatment image data; and
wherein the image acquisition device (5) is configured to provide the treatment image data.

12. System according to claim 10 or 11, wherein the foldable implant (4) is a foldable valve implant.

13. A method for predicting the position of an unfolded state of a foldable implant in cardiovascular tissue, the method comprising the following steps:
a) receiving (101) pre-treatment 3D planning image data with a receiving unit;
b) receiving (102) treatment image data with the receiving unit, the treatment image data comprising catheter image data with landmark image data;
c) segmenting (103) the pre-treatment 3D planning image data with a segmentation module resulting in segmented 3D planning image data;
d) locating (104) the landmark image data within the treatment image data with a search unit resulting in a known landmark position and a known landmark orientation; and
e) registering (105) the segmented 3D planning image data to the treatment image data with a registration module;
f) simulating (106) an unfolding implant relative to the known landmark position and the known landmark orientation in the cardiovascular tissue with a simulation unit resulting in a simulated unfolded implant positioned in cardiovascular tissue; and
wherein step b) and c) may be performed in any order.

14. A computer program element for controlling an apparatus according to one of the claims 1 to 9 or a system according to one of claim 10 to 12, which, when being executed by a processing unit, is adapted to perform the method steps of claim 13.

15. A computer readable medium having stored the program element of claim 14.
